(19) Europäisches Patentamt
European Patent Office
Office européen des brevets

(11) EP 0 718 307 A2

(12) **DEMANDE DE BREVET EUROPEEN**

(43) Date de publication:
**26.06.1996 Bulletin 1996/26**

(51) Int Cl.⁶: **C07K 5/078**, A61K 38/05, C07C 309/51, C07C 309/88

(21) Numéro de dépôt: **95402840.3**

(22) Date de dépôt: **18.12.1995**

(84) Etats contractants désignés:
**AT BE CH DE DK ES FR GB GR IE IT LI LU NL PT SE**

(30) Priorité: **23.12.1994 FR 9415549**

(71) Demandeur: **SYNTHELABO**
**F-92350 Le Plessis Robinson (FR)**

(72) Inventeurs:
- **Altenburger, Jean Michel**
**F-92190 Meudon (FR)**
- **Lassalle, Gilbert**
**F-92140 Clamart (FR)**

(74) Mandataire: **Thouret-Lemaitre, Elisabeth et al**
**SYNTHELABO,**
**Service Brevets,**
**B.P. 72**
**92352 Le Plessis-Robinson Cédex (FR)**

(54) **Dérivés de 1-oxo-2-(phénylsulfonylamino)pentylpipéridine, leur préparation et leur application en thérapeutique**

(57) Composés répondant à la formule (I)

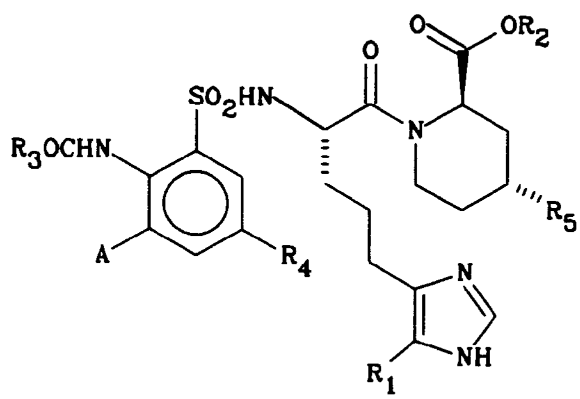

(I)

dans laquelle $R_1$ représente soit un atome d'hydrogène, soit un groupe $(C_1\text{-}C_4)$alkyle, $R_2$ représente soit un atome d'hydrogène, soit un groupe $(C_1\text{-}C_4)$alkyle droit ou ramifié, $R_3$ représente soit un groupe $(C_1\text{-}C_7)$alkyle droit ou ramifié, soit un groupe $-(CH_2)_nOCH_3$ (où n est 1, 2 ou 3), soit un groupe $-CH_2O(C_2H_4O)_mCH_3$ (où m est 1, 2 ou 3), $R_4$ représente soit un atome d'hydrogène, soit un atome d'halogène, $R_5$ représente un groupe $(C_1\text{-}C_4)$alkyle droit ou ramifié et A représente soit un groupe phényle éventuellement substitué par un ou plusieurs substituants choisis parmi les atomes d'halogènes et les groupes $(C_1\text{-}C_4)$alkyle droit ou ramifié, $(C_1\text{-}C_4)$alcoxy droit ou ramifié et trifluorométhyle, soit un hétérocycle choisi parmi les groupes pyridinyle, thiényle, furyle, éventuellement substitués comme ci-dessus, soit un groupe cyclo$(C_5\text{-}C_8)$alkyle,
sous forme de base libre ou de sels d'addition à des acides pharmaceutiquement acceptables.

Application en thérapeutique.

EP 0 718 307 A2

## Description

La présente invention a pour objet des dérivés de 1-oxo-2-(phénylsulfonylamino)pentylpipéridine, leur préparation et leur application en thérapeutique.

Les composés de l'invention répondent à la formule (I)

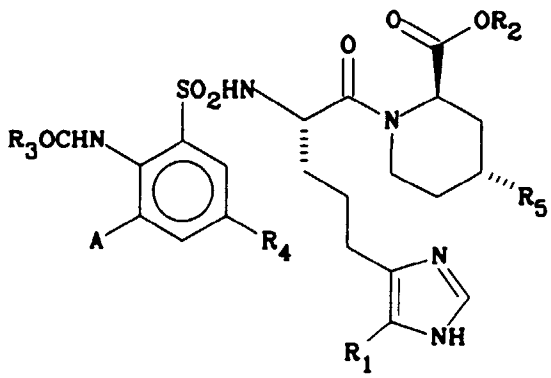

(I)

dans laquelle

$R_1$ représente soit un atome d'hydrogène, soit un groupe $(C_1\text{-}C_4)$ alkyle,
$R_2$ représente soit un atome d'hydrogène, soit un groupe $(C_1\text{-}C_4)$alkyle droit ou ramifié,
$R_3$ représente soit un groupe $(C_1\text{-}C_7)$alkyle droit ou ramifié, soit un groupe $-(CH_2)_nOCH_3$ (où n est 1, 2 ou 3), soit un groupe $-CH_2O(C_2H_4O)_mCH_3$ (où m est 1, 2 ou 3),
$R_4$ représente soit un atome d'hydrogène, soit un atome d'halogène,
$R_5$ représente un groupe $(C_1\text{-}C_4)$alkyle droit ou ramifié et A représente soit un groupe phényle éventuellement substitué par un ou plusieurs substituants choisis parmi les atomes d'halogènes et les groupes $(C_1\text{-}C_4)$alkyle droit ou ramifié, $(C_1\text{-}C_4)$alcoxy droit ou ramifié et trifluorométhyle, soit un hétérocycle choisi parmi les groupes pyridinyle, thiényle, furyle, éventuellement substitués comme ci-dessus, soit un groupe cyclo$(C_5\text{-}C_8)$alkyle.

Les composés préférés selon l'invention sont ceux pour lesquels
$R_3$ représente un groupe $(C_1\text{-}C_7)$alkyle droit ou ramifié et A représente un hétérocycle choisi parmi les groupes pyridinyle, thiényle, furyle, éventuellement substitués par un ou plusieurs substituants choisis parmi les atomes d'halogènes et les groupes $(C_1\text{-}C_4)$alkyle droit ou ramifié, $(C_1\text{-}C_4)$alcoxy droit ou ramifié et trifluorométhyle.

Les composés de l'invention possèdent 3 centres asymétriques. La configuration préférentielle du groupe pipéridinyle est [2*R*, 4*R*].

La configuration de la partie acide aminé centrale

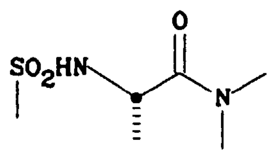

est [*S*].

Les composés peuvent exister sous forme de bases libres ou de sels d'addition à des acides pharmaceutiquement acceptables.

Toutes ces formes font partie de l'invention.

Dans les schémas qui suivent, le groupe $-CPh_3$ représente le groupe triphénylméthyle.

Selon l'invention les composés de formule (I) peuvent être synthétisés selon le schéma 1.

On fait réagir un acide sulfonique de formule (II) dans laquelle A et $R_4$ sont tels que définis précédemment avec un chlorure d'acide de formule (III) dans laquelle $R_3$ est tel que défini précédemment, dans un solvant aprotique tel que le dichlorométhane en présence d'une base comme la pyridine, puis on ajoute de la triéthylamine en excès pour obtenir un composé de formule (IV) sous forme de sel de triéthylamine ; ensuite on fait réagir le composé de formule (IV) avec de l'anhydride trifluoracétique et on obtient un composé de formule (V) à partir duquel on prépare un composé de formule (VI) par action du pentachlorure de phosphore dans un solvant tel que le dichlorométhane ; enfin on fait réagir le composé de formule (VI) avec le chlorhydrate de l'amine de formule (VII) (dans laquelle $R_1$ représente un atome d'hydrogène ou un

Schéma 1

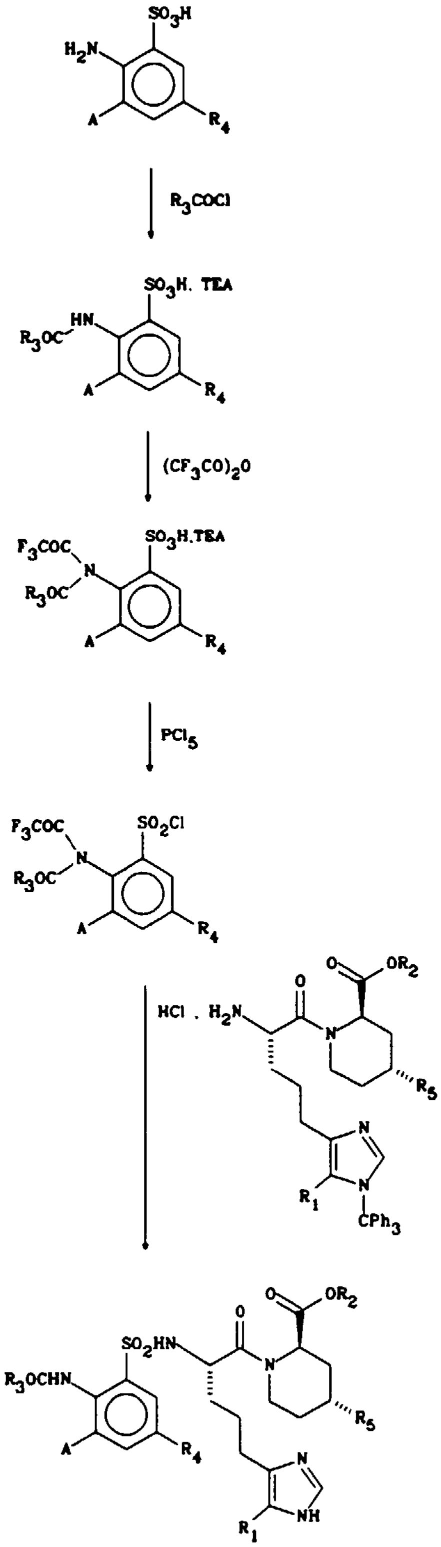


(II)

(III)

(IV)

(V)

(VI)

(VII)

(I)

groupe ($C_1$-$C_4$)alkyle, $R_2$ représente un groupe ($C_1$-$C_4$)alkyle droit ou ramifié et $R_5$ représente un groupe ($C_1$-$C_4$)alkyle droit ou ramifié) dans un solvant aprotique tel que le dichlorométhane en présence d'une base comme la triéthylamine

puis on déprotège le noyau imidazolyle et on enlève le résidu trifluoroacétyle dans un mélange acide acétique:éthanol ou acide acétique:tétrahydrofurane:eau au reflux et on obtient un composé de formule (I) (dans laquelle $R_2$ représente un groupe ($C_1$-$C_4$)alkyle droit ou ramifié). Dans le cas où l'on souhaite obtenir un composé de formule (I) dans laquelle $R_2$ représente un atome d'hydrogène, alors on réalise une saponification du composé de formule (I) correspondant (dans laquelle $R_2$ représente un groupe ($C_1$-$C_4$)alkyle droit ou ramifié) dans des conditions classiques connues de l'homme du métier.

Dans une variante du procédé, illustrée dans le schéma 2, pour préparer les composés de formule (Ia) (dans laquelle $R'_3$ représente un groupe ($C_1$-$C_7$)alkyle droit ou ramifié), on peut faire réagir un composé de formule (II) avec de la triéthylamine pour former un sel de triéthylamine que l'on fait réagir avec un chlorure d'acide de formule (IIIa) ou avec un anhydride de formule (IIIb) et on obtient un imide symétrique sous forme de sel de triéthylamine de formule (Va), sel que l'on traite par du pentachlorure de phosphore pour obtenir un composé de formule (VIa) sur lequel on fait réagir le chlorhydrate de l'amine de formule (VII) dans un solvant aprotique tel que le dichlorométhane en présence d'une base comme la triéthylamine puis on déprotège le noyau imidazolyle et on enlève un des résidus $-COR'_3$ dans un mélange acide acétique:éthanol, acide acétique:eau ou acide acétique:tétrahydrofurane:eau au reflux et on obtient un composé de formule (Ia) (dans laquelle $R_2$ représente un groupe ($C_1$-$C_4$)alkyle droit ou ramifié).

Dans le cas où l'on souhaite obtenir un composé de formule (Ia) dans laquelle $R_2$ représente un atome d'hydrogène, alors on réalise une saponification du composé de formule (Ia) correspondant (dans laquelle $R_2$ représente un groupe

Schéma 2

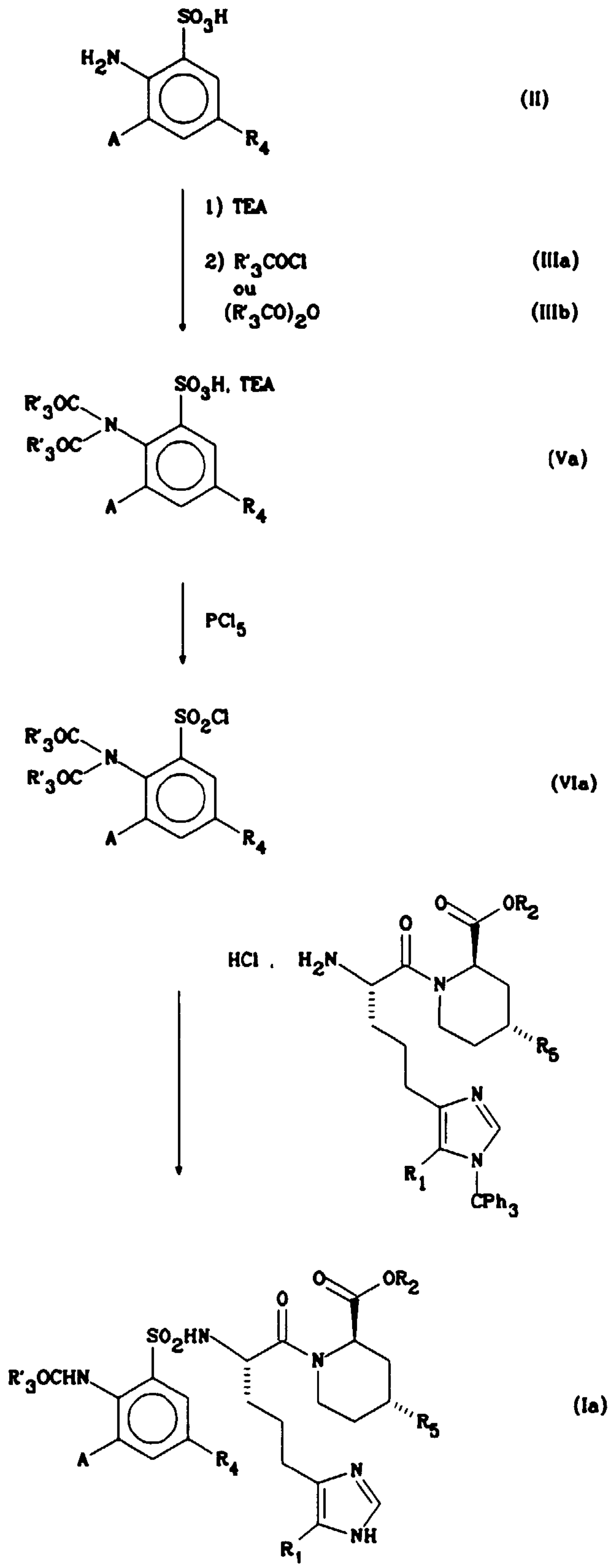


(II)

(IIIa)

(IIIb)

(Va)

(VIa)

(VII)

(Ia)

($C_1$-$C_4$)alkyle droit ou ramifié) dans des conditions classiques connues de l'homme du métier.

On peut également utiliser le procédé illustré par le schéma 3.

On traite le composé de formule (IIb) dans laquelle $R_4$ représente un atome d'halogène avec de la triéthylamine et on fait réagir le sel ainsi obtenu avec un chlorure d'acide de formule (IIIa) dans laquelle $R'_3$ est tel que défini précédemment et on obtient un composé de formule (IVb) à partir duquel on prépare un composé de formule (VIb) par action du pentachlorure de phosphore dans un solvant tel que le dichlorométhane puis on fait réagir le composé (VIb) avec le chlorhydrate de l'amine de formule (VII) (dans laquelle $R_1$ représente un atome d'hydrogène ou un groupe ($C_1$-$C_4$) alkyle, $R_2$ représente un groupe ($C_1$-$C_4$)alkyle droit ou ramifié et $R_5$ représente un atome d'hydrogène ou un groupe ($C_1$-$C_4$)alkyle droit ou ramifié) dans un solvant aprotique tel que le dichlorométhane puis on enlève un résidu -COR'$_3$ par un traitement à l'ammoniac dans un solvant aprotique et on obtient un composé de formule (VIII) que l'on fait réagir avec un composé de formule (IX) (dans laquelle A est tel que défini précédemment et R est un groupe ($C_1$-$C_4$)alkyle) dans un solvant comme le diméthylformamide en présence d'un catalyseur comme le tétrakis(triphénylphosphine) palladium (0) pour former un composé de formule (X) que l'on chauffe à la température de reflux en milieu acide, par exemple dans un mélange acide acétique:eau, et on obtient un composé de formule (Ia) dans laquelle $R_4$ représente un atome d'halogène. Si l'on souhaite obtenir un composé de formule (Ia) dans laquelle $R_4$ est un atome d'hydrogène, alors on soumet le composé de formule (Ia) correspondant (dans laquelle $R_4$ est un atome d'halogène) à une hydrogénolyse.

Dans le cas où l'on souhaite obtenir un composé de formule (Ia) dans laquelle $R_2$ représente un atome d'hydrogène, alors on réalise une saponification du composé de formule (Ia) correspondant (dans laquelle $R_2$ représente un groupe ($C_1$-$C_4$)alkyle droit ou ramifié) dans des conditions classiques connues de l'homme du métier.

Schéma 3

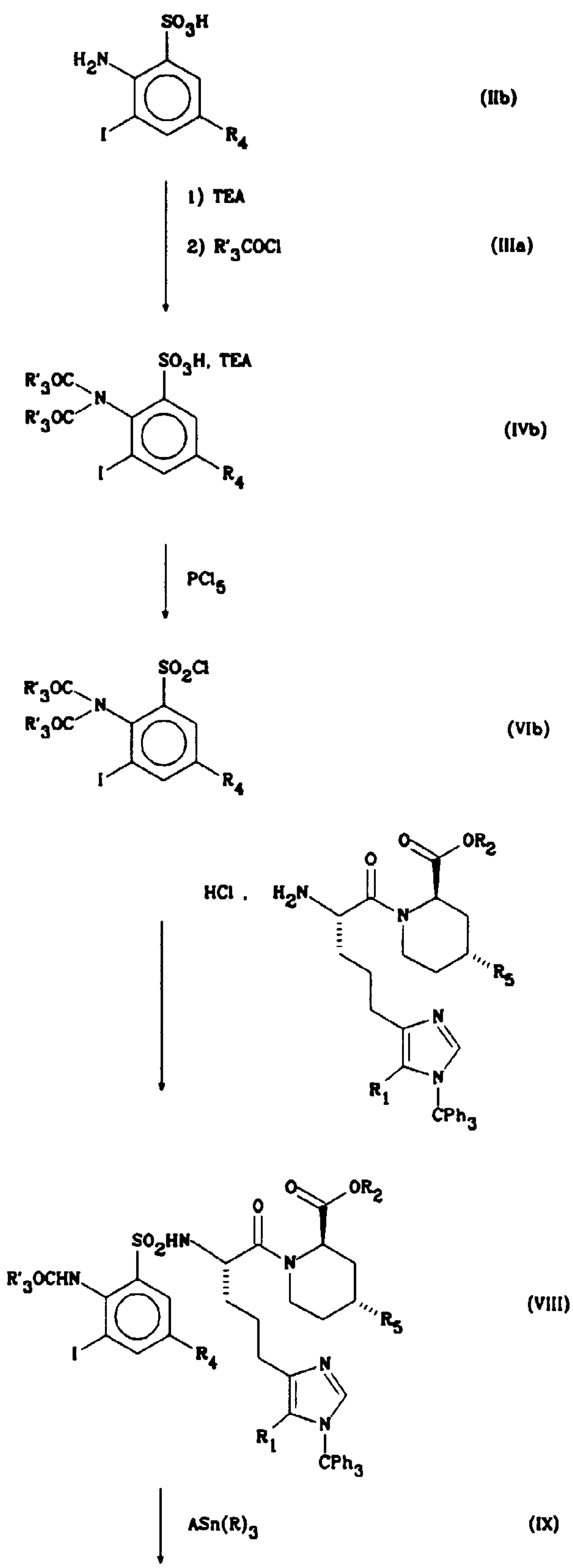


(IIb)

(IIIa)

(IVb)

(VIb)

(VII)

(VIII)

(IX)

Schéma 3 (suite)

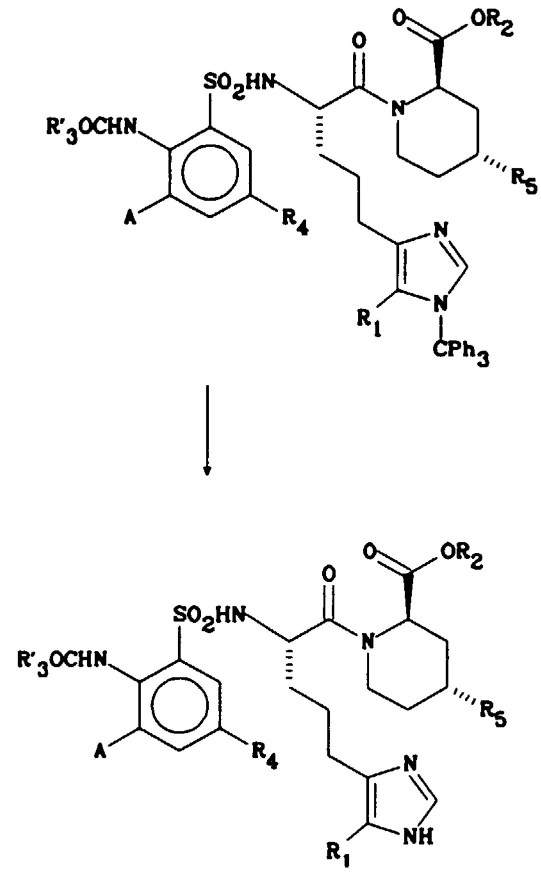

(X)

(Ia)

Pour les composés de formule (I) dans laquelle $R_3$ représente un groupe $-(CH_2)_nOCH_3$ (où n est 1, 2 ou 3), ou $-CH_2O(C_2H_4O)_mCH_3$ (où m est 1, 2 ou 3), on peut également préparer à partir du composé de formule (IIb) un imide mixte par une méthode analogue à celle décrite pour le composé de formule (V) du schéma 1, puis on continue selon le schéma 3 en faisant réagir cet imide mixte avec l'amine de formule (VII).

Les composés intermédiaires de formule (XI)

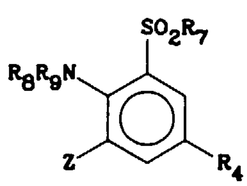

(XI)

dans laquelle soit $R_8$ représente un atome d'hydrogène ou un groupe trifluoroacétyle, $R_9$ un groupe $-COR_3$ (où $R_3$ est tel que défini précédemment) et $R_7$ un groupe hydroxy, soit $R_8$ représente un groupe trifluoroacétyle, $R_9$ un groupe $-COR_3$ et $R_7$ un atome de chlore, soit $R_8$ et $R_9$ représentent chacun un groupe $-COR'_3$ (où $R'_3$ est tel que défini précédemment) et $R_7$ un atome de chlore ou un groupe hydroxy,
Z représente un atome d'iode ou un groupe phényle éventuellement substitué par un ou plusieurs substituants choisis parmi les atomes d'halogènes et les groupes $(C_1\text{-}C_4)$alkyle droit ou ramifié, $(C_1\text{-}C_4)$alcoxy droit ou ramifié et trifluorométhyle, soit un hétérocycle choisi parmi les groupes pyridinyle, thiényle, furyle, éventuellement substitués comme ci-dessus, soit un groupe cyclo$(C_5\text{-}C_8)$alkyle et
$R_4$ est tel que défini précédemment,
sont nouveaux et font également partie de l'invention.

Les composés de départ sont disponibles dans le commerce ou décrits dans la littérature ou peuvent être préparés selon des méthodes qui y sont décrites ou qui sont connues de l'homme du métier.

Les composés de formule (II) et leur préparation sont décrits dans la demande de brevet français no FR 9414129. Les composés de formule (VII) sont décrits dans la demande de brevet européen no EP 0643047.

Les exemples suivants illustrent la préparation de certains composés conformément à l'invention. Les microanalyses et les spectres IR et RMN confirment la structure des composés obtenus. Les numéros des composés exemplifiés renvoient à ceux du tableau donné plus loin qui illustre les structures chimiques et les propriétés physiques de quelques composés selon l'invention .

Exemple 1 (composé no 8)

chlorhydrate de [2*R*-[1(*S*), 2α, 4β]]-4-éthyl-1-[5-(1*H*-imidazol-4-yl)-2-[[[2-[(3-méthoxy-1-oxopropyl)amino][1,1'-biphényl]-3-yl]sulfonyl]amino]-1-oxopentyl]pipéridine-2-carboxylate d'éthyle

1.1. sel de *N,N*-diéthyléthanamine de l'acide 2-[(3-méthoxy-1-oxopropyl)amino][1,1'-biphényle]-3-sulfonique

A une solution de 1,74 g (7 mmoles) d'acide 2-amino[1,1'-biphényle]-3-sulfonique et de 1,6 ml (19,5 mmoles) de pyridine dans 7 ml de dichlorométhane, on ajoute goutte à goutte à 0 °C sous atmosphère d'azote une solution de 1,3 g (10,5 mmoles) de chlorure de 3-méthoxypropionyle dans 3 ml de dichlorométhane. On laisse le milieu réactionnel sous agitation pendant 2 heures à 0 °C, on ajoute de la triéthylamine et du méthanol en excès, puis on concentre sous pression réduite. On purifie le résidu par chromatographie sur colonne de gel de silice en éluant par un mélange méthanol:dichlorométhane:triéthylamine (2:98:0,001).
On obtient 2,3 g de produit sous forme d'une huile visqueuse que l'on utilise telle quelle dans l'étape suivante.
Rendement = 100 %

1.2. sel de N,N-diéthyléthanamine de l'acide 2-[(3-méthoxy-1-oxopropyl) (trifluoroacétyl)amino][1,1'-biphényle]-3-sulfonique

On chauffe pendant 3 heures à la température de reflux un mélange de 2,3 g (7 mmoles) de sel de N,N-diéthyléthanamine de l'acide 2-[(3-méthoxy-1-oxopropyl)amino][1,1'-biphényle]-3-sulfonique et de 9,8 ml (70 mmoles) d'anhydride trifluoracétique puis on concentre le milieu réactionnel sous pression réduite.
On obtient 3,8 g de produit sous forme d'une huile visqueuse que l'on utilise telle quelle dans l'étape suivante.
Rendement = 100 %

1.3. chlorure de 2-[(3-méthoxy-1-oxopropyl) (trifluoroacétyl)amino] [1,1'-biphényle]-3-sulfonyle

A une solution de 3,8 g (7 mmoles) de sel de *N,N*-diéthyléthanamine de l'acide 2-[(3-méthoxy-1-oxopropyl) (trifluoroacétyl)amino][1,1'-biphényle]-3-sulfonique dans 14 ml de dichlorométhane, on ajoute 1,75 g (8,4 mmoles) de pentachlorure de phosphore, on chauffe le mélange pendant 3 heures à la température de reflux puis on concentre sous pression réduite. On purifie le résidu ainsi obtenu par chromatographie sur colonne de gel de silice en éluant par du dichlorométhane.
On obtient 1,3 g de produit sous forme d'une huile visqueuse que l'on utilise tel quel dans l'étape suivante.
Rendement = 40 %
RMN ($CDCLl_3$), 200 Mhz, 6, (ppm): 8,4-8,2 (m, 1H) ; 7,8-7,6 (m, 2H) ; 7,5-7,3 (m, 3H) ; 7,3-7,2 (m, 2H) ; 3,8-3,5 (m, 2H) ; 3,3 (s, 3H) ; 3,0-2,7 (m, 2H)

1.4. chlorhydrate de [2R-[1(*S*), 2α, 4β]]-4-éthyl-1-[5-(1*H*-imidazol-4-yl)-2-[[[2-[(3-méthoxy-1-oxopropyl)amino] [1,1'-biphényl]-3-yl]sulfonyl]amino]-1-oxopentyl] pipéridine-2-carboxylate d'éthyle

A un mélange de 1,5 g (2,4 mmoles) de chlorhydrate de [2*R*-[1(*S*), 2α, 4β]]-1-[2-amino-1-oxo-5-[1-(triphénylméthyl)-1*H*-imidazol-4-yl]pentyl]-4-éthylpipéridine-2-carboxylate d'éthyle et de 0,8 ml (5,8 mmoles) de triéthylamine dans 15 ml de dichlorométhane, on ajoute goutte à goutte, à 0 °C sous atmosphère d'azote, 1,3 g (2,89 mmoles) de chlorure de 2-[(3-méthoxy-2-propanoyl) (trifluoroacétyl)] amino[1,1'-biphényle]-3-sulfonyle en solution dans 3 ml de dichlorométhane. On laisse le milieu réactionnel sous agitation à cette température pendant 6 heures puis on le concentre sous pression réduite. On reprend le résidu dans 50 ml d'acétate d'éthyle puis on le lave successivement par 50 ml d'une solution aqueuse d'acide chlorhydrique 0,5 N, puis par 50 ml d'une solution d'hydrogénocarbonate de sodium à 5 % puis par 50 ml d'une solution saturée de chlorure de sodium et on sèche sur sulfate de magnésium. On chauffe le résidu ainsi obtenu à 90 °C pendant 2 heures dans un mélange contenant 50 ml d'acide acétique et 50 ml d'éthanol puis on concentre sous pression réduite. On purifie ce dernier résidu par chromatographie sur colonne de gel de silice en éluant par un mélange méthanol:dichlorométhane (5:95).

On obtient 1,2 g de produit sous forme de base.
Rendement = 75 %
On prépare le chlorhydrate en dissolvant 1,2 g (1,8 mmoles) de base dans 36 ml d'une solution d'isopropanol dans l'acide chlorhydrique 0,1 N et en évaporant sous pression réduite.
Point de fusion = 74 °C
$[\alpha]_D^{20}$ = + 60 ° (c = 0,2 ; méthanol)

Exemple 2 (composé no 15)

chlorhydrate de [2*R*-[1(*S*), 2α, 4β]]-4-éthyl-1-[5-(5-méthyl-1*H*-imidazol-4-yl)-2-[[[3'-méthyl-2-[(1-oxopropyl)amino][1,1'-biphényl]-3-yl]sulfonyl]amino]-1-oxopentyl]pipéridine-2-carboxylate d'éthyle

2.1. sel de *N,N*-diéthyléthanamine de l'acide 2-[bis(1-oxopropyl)amino]-3'-méthyl[1,1'-biphényle]-3-sulfonique

On dissout 2,1 g (8 mmoles) d'acide 2-amino-3'méthy1[1,1'-biphényle]-3-sulfonique dans 10 ml de dichlorométhane, on ajoute 1,34 ml (9,6 mmoles) de triéthylamine et on évapore à sec. On dissout alors le sel obtenu dans 15,5 ml d'anhydride propionique, on chauffe le mélange pendant 8 heures à 100 °C et on évapore à sec. On purifie le résidu par chromatographie sur colonne de gel de silice en éluant par un mélange dichlorométhane/éthanol/triéthylamine (98:2:0,001).
On obtient 3,6 de produit sous forme d'une huile visqueuse que l'on utilise telle quelle dans l'étape suivante.
Rendement = 95 %

2.2. chlorure de 2-[bis(1-oxopropyl)amino]-3'méthyl[1,1'-biphényle]-3-sulfonyle

A une solution de 3,6 g (7,6 mmoles) de sel de N,N-diéthyléthanamine de l'acide 2-[bis(1-oxopropyl)amino]-3'méthyl[1,1'-biphényle]-3-sulfonique dans 8 ml de dichlorométhane, à 0 °C, on ajoute 1,6 g (7,6 mmoles) de pentachlorure de phosphore et on laisse la température du mélange revenir à la température ambiante. On chauffe ensuite le mélange pendant 4 heures à la température de reflux puis on laisse la température revenir à l'ambiante. On ajoute de l'éther, on filtre, on concentre le filtrat et on purifie le résidu sur colonne de florisil™ en éluant par un mélange éther/pentane (1:1). On recristallise dans un mélange éther/pentane.
On obtient 1,7 g de produit sous forme de cristaux blanchâtres.
Rendement = 58 %
Point de fusion = 91 °C

2.3. [2*R*-[1(*S*), 2α, 4β]]-1-[2-[[[2-[bis(1-oxopropyl)amino]-3'méthyl[1,1'-biphényl]-3-yl]sulfonyl]amino]-5-[5-méthyl-1-(tripphény1méthyl)-1H-imidazo1-4-yl]-1-oxopentyl]-4-éthylpipéridine-2-carboxylate d'éthyle

On dissout dans 8 ml de dichlorométhane 787 mg (2 mmoles) de chlorure de 2-[bis(1-oxopropyl)amino]-3'méthyl[1,1'-biphényle]-3-sulfonyle et on ajoute, à 0 °C, 1,3 g (2 mmoles) de chlorhydrate de [2*R*-[1(*S*), 2α, 4β]]-1-[2-amino-5-[5-méthyl-1-(triphénylméthyl)-1*H*-imidazol-4-yl]-1-oxopentyl]-4-éthylpipéridine-2-carboxylate d'éthyle et 0,9 ml de triéthylamine. On laisse la température du mélange revenir à la température ambiante, on évapore et on reprend le résidu dans 100 ml d'acétate d'éthyle. On lave successivement par 50 ml d'une solution d'acide chlorhydrique 1 N, 50 ml d'une solution saturée d' hydrogénocarbonate de sodium et 50 ml d'une solution saturée de chlorure de sodium, on sèche sur sulfate de magnésium puis on évapore à sec.
On obtient 1,6 g de produit que l'on utilise tel quel dans l'étape suivante.
Rendement = 84 %

2.4. chlorhydrate de [2*R*-[1(*S*), 2α, 4β]]-4-éthyl-1-[5-(5-méthyl-1*H*-imidazol-4-yl)-2-[[[3'-méthyl-2-[(1-oxopropyl) amino][1,1'-biphényl]-3-yl]sulfonyl]amino]-1-oxopentyl] pipéridine-2-carboxylate d'éthyle

On dissout 1,6 g (1,7 mmoles) de [2*R*-[1(*S*), 2α, 4β]]-1-[2-[[[2-[bis(1-oxopropyl)amino]-3'méthyl[1,1'-biphényl]-3-yl]sulfonyl]amino]-5-[5-méthyl-1-(triphénylméthyl)-1*H*-imidazol-4-yl]-1-oxopentyl]-4-éthylpipéridine-2-carboxylate d'éthyle dans 85 ml d'acide acétique et 17 ml d'eau et on chauffe à la température de reflux pendant 6 heures. On évapore à sec, on reprend le résidu dans 100 ml d'acétate d'éthyle et on lave successivement par 20 ml d'une solution saturée d'hydrogénocarbonate de sodium et 10 ml d'une solution saturée de chlorure de sodium puis on évapore à sec. On purifie le résidu par chromatographie sur colonne de gel de silice en éluant par un mélange dichlorométhane/éthanol (95:5).
On obtient 1 g de produit sous forme de base.

On prépare le chlorhydrate dans une solution d'isopropanol dans l'acide chlorhydrique 0,1 N et on concentre de nouveau sous pression réduite. On purifie ce dernier résidu sur colonne RP 18 en éluant par un mélange acétonitrile:eau (1:1).
Après lyophylisation on obtient 893 mg de produit.
Rendement = 67 %
Point de fusion = 140 °C
$[a]_D^{20}$ = + 115 ° (c = 0,2 ; méthanol)

Exemple 3 (composé no 3)

chlorhydrate de [2*R*-[1(*S*), 2α, 4β]]-4-éthyl-1-[5-(5-méthyl-1*H*-imidazol-4-yl)-1-oxo-2-[[[2-[(1-oxopropyl)amino][1,1'-biphényl]-3-yl]sulfonyl]amino]pentyl]pipéridine-2-carboxylate d'éthyle

3.1. chlorure de 2-[bis(1-oxopropyl)amino][1,1'-biphényle]-3-sulfonyle

On prépare le produit selon la méthode décrite dans l'exemple 2 à partir de l'acide 2-amino-[1,1'-biphényle]-3-sulfonique et de l'anhydride propionique.
Point de fusion = 113,8 °C

3.2. [2*R*-[1(*S*), 2α, 4β]]-1-[2-[[[2-[bis(1-oxopropyl)amino] [1,1'-biphényl]-3-yl]sulfonyl]amino]-5-[5-méthyl-1-(triphénylméthyl)-1*H*-imidazol-4-yl]-1-oxopentyl]-4-éthylpipéridine-2-carboxylate d'éthyle

On dissout 1,88 g (4,85 mmoles) de chlorure de 2-[bis(1-oxopropyl)amino][1,1'-biphényle]-3-sulfonyle dans 20 ml de dichlorométhane et on place le mélange à 0 °C. On ajoute alors 2,99 g (4,5 mmoles) de chlorhydrate de [2*R*-[1(*S*), 2α, 4β]]-1-[2-amino-5-[5-méthyl-1-(triphénylméthyl)-1*H*-imidazol-4-yl]-1-oxopentyl]-4-éthylpipéridine-2-carboxylate d'éthyle et goutte à goutte 1,6 ml de triéthylamine. On laisse sous agitation à cette température pendant une nuit. On concentre sous pression réduite, on reprend le résidu par 100 ml d'acétate d'éthyle et on lave successivement par 50 ml d'une solution d'acide chlorhydrique 1 N, par 50 ml d'une solution saturée en hydrogénocarbonate de sodium puis par 50 ml d'une solution saturée en chlorure de sodium. On sèche sur sulfate de magnésium et on concentre sous pression réduite. On purifie le résidu par chromatographie sur colonne de gel de silice en éluant par un mélange dichlorométhane/méthanol (98:2).
On obtient 2,9 g de produit.
Rendement = 62 %

3.3. chlorhydrate de [2*R*-[1(*S*), 2α, 4β]]-4-éthyl-1-[5-(5-méthyl-1*H*-imidazol-4-yl)-1-oxo-2-[[[2-[(1-oxopropyl) amino][1,1'-biphényl]-3-yl]sulfonyl]amino]pentyl] pipéridine-2-carboxylate d'éthyle

A 2,9 g (3 mmoles) de [2*R*-[1(*S*), 2α, 4β]]-1-[2-[[[2-[bis(1-oxopropyl)amino] [1,1'-biphényl]-3-yl]sulfonyl]amino]-5-[5-méthyl-1-(triphénylméthyl)-1*H*-imidazol-4-yl]-1-oxopentyl]-4-éthylpipéridine-2-carboxylate d'éthyle, on ajoute 150 ml d'acide acétique et 50 ml d'eau et on chauffe le mélange à la température de reflux pendant 6,5 heures. On évapore à sec et on purifie le résidu par chromatographie sur colonne de gel de silice en éluant par un mélange dichlorométhane/méthanol (95:5).
On obtient 1,7 g de produit sous forme de base. On prépare le chlorhydrate dans 10 ml d'une solution d'isopropanol dans l'acide chlorhydrique 0,1 N et on concentre de nouveau sous pression réduite. On purifie ce dernier résidu sur colonne RP 18 en éluant par un mélange acétonitrile:eau (1:1).
On obtient 1,3 g de produit sous forme de chlorhydrate.
Rendement = 63 %
Point de fusion = 104-105 °C
$[a]_D^{20}$ = + 105 ° (c = 0,2 ; méthanol)

Exemple 4 (composé no 29)

chlorhydrate de [2*R*-[1(*S*), 2α, 4β]]-4-éthyl-1-[5-(5-méthyl-1*H*-imidazol-4-yl)-1-oxo-2-[[[2-[(1-oxopropyl)amino]-3-pyridin-2-ylphényl]sulfonyl]amino]pentyl]pipéridine-2-carboxylate d'éthyle

4.1. sel de *N,N*-diéthyléthanamine de l'acide 2-[bis(1-oxopropyl)amino]-5-bromo-3-iodobenzènesulfonique

On chauffe pendant 16 heures à la température de reflux une solution de 31,7 g (66 mmoles) de sel de N,N-

diéthyléthanamine de l'acide 2-amino-5-bromo-3-iodobenzènesulfonique dans 114 ml (1,32 mole) de chlorure de propionyle, puis on concentre le milieu réactionnel sous pression réduite et on cristallise le produit dans 100 ml d'acétate d'éthyle.
On obtient 32,5 g de produit sous forme de cristaux blancs.
Rendement = 84 %
Point de fusion = 124-128 °C

4.2. chlorure de 2-[bis(1-oxopropyl)amino]-5-bromo-3-iodobenzènesulfonyle

A une solution de 32,5 g (55 mmoles) de sel de *N,N*-diéthyléthanamine de l'acide 2-[bis(1-oxopropyl) amino]-5-bromo-3-iodobenzènesulfonique dans le dichlorométhane, on ajoute par petites quantités, à 0 °C sous azote, 16,8 g (82,4 mmoles) de pentachlorure de phosphore. On laisse la température revenir à la température ambiante et on chauffe le mélange pendant 4 heures à la température de reflux. On verse 200 ml d'éther, on filtre et on concentre le filtrat. On purifie le résidu par chromatographie sur colonne de florisil™ en éluant par un mélange éther/hexane (2:8). On recristallise le produit dans un mélange éther/pentane.
On obtient 12 g de produit sous forme de cristaux blancs.
Rendement = 43 %
Point de fusion = 127-132 °C

4.3. [2*R*-[1(*S*), 2α, 4β]]-1-[2-[[[5-bromo-3-iodo-2-[(1-oxopropyl)amino]phényl]sulfonyl]amino]-5-[5-méthyl-1-(triphénylméthyl)-1*H*-imidazol-4-yl]-1-oxopentyl]-4-éthylpipéridine-2-carboxylate d'éthyle

A une solution de 1,28 g (2 mmoles) de chlorhydrate de [2*R*-[1(*S*), 2α, 4β]]-1-[2-amino-5-[5-méthyl-1-(triphénylméthyl)-1*H*-imidazol-4-yl]-1-oxopentyl]-4-éthylpipéridine-2-carboxylate d'éthyle dans du dichlorométhane, on ajoute successivement à 0 °C sous azote, 1,02 g (2 mmoles) de chlorure de 2-[bis(1-oxopropyl)amino]-5-bromo-3-iodobenzènesulfonyle puis goutte à goutte 0,69 µl (5 mmoles) de triéthylamine. On laisse le milieu réactionnel pendant 6 heures sous agitation à 0 °C puis on le reprend par 100 ml d'acétate d'éthyle. Ensuite on le lave successivement avec 2 fois 50 ml d'acide chlorhydrique 0,5 N, 50 ml d'une solution saturée d'hydrogénocarbonate de sodium et 50 ml d'une solution saturée en chlorure de sodium. Finalement on sèche sur sulfate de magnésium et on concentre sous pression réduite. On obtient 2,1 g de produit sous forme d'une huile visqueuse que l'on reprend dans 100 ml de tétrahydrofurane et que l'on traite à 0 °C par de l'ammoniac gaz. On laisse le milieu réactionnel sous agitation pendant 2 heures à cette température et on concentre sous pression réduite. On purifie le résidu par chromatographie sur colonne de gel de silice en éluant par un mélange dichlorométhane/méthanol (98:2).
On obtient 1,4 g de produit sous forme d'une huile visqueuse que l'on utilise telle quelle dans l'étape suivante.
Rendement = 70 %

4.4. [2*R*-[1(*S*), 2α, 4β]]-1-[2-[[[5-bromo-2-[(1-oxopropyl) amino]-3-pyridin-2-ylphényl]sulfonyl]amino]-5-[5-méthyl-1-(triphénylméthyl)-1*H*-imidazol-4-yl]-1-oxopentyl]-4-éthylpipéridine-2-carboxylate d'éthyle

On chauffe à 95 °C sous argon pendant 4 heures un mélange contenant 1,2 g (1,2 mmoles) de [2*R*-[1(*S*), 2α, 4β]]-1-[2-[[[5-bromo-3-iodo-2-[(1-oxopropyl)amino]phényl]sulfonyl] amino]-5-[5-méthyl-1-(triphény1méthyl)-1*H*-imidazol-4-yl]-1-oxopentyl]-4-éthylpipéridine-2-carboxylate d'éthyle, 0,53 g (1,44 mmoles) de 2-(tributylstannyl)pyridine, 14 mg (0,07 mmole) de iodure de cuivre et 83 mg (0,07 mmole) de tétrakis(triphénylphosphine) palladium (0) dans 2,4 ml de diméthylformamide. Ensuite on reprend le milieu réactionnel par 100 ml d'acétate d'éthyle, on le lave par 2 fois 100 ml d'une solution d'hydrogénocarbonate de sodium à 5 % puis par 50 ml d'une solution saturée en chlorure de sodium, on sèche sur sulfate de magnésium et on concentre sous pression réduite. On purifie le résidu par chromatographie sur colonne de gel de silice en éluant par un mélange dichlorométhane/méthanol (98:2).
On obtient 0,72 g de produit que l'on utilise tel quel dans l'étape suivante.
Rendement = 63 %
Point de fusion = 90-94 °C

4.5. [2*R*-[1(*S*), 2α, 4β]]-1-[2-[[[5-bromo-2-[(1-oxopropyl) amino]-3-pyridin-2-ylphényl]sulfonyl]amino]-5-[5-méthyl--1*H*-imidazol-4-yl]-1-oxopentyl]-4-éthylpipéridine-2-carboxylate d'éthyle

On chauffe à 100 °C pendant 1 heure 0,71 g (0,75 mmole) de [2*R*-[1(*S*), 2α, 4β]]-1-[2-[[[5-bromo-2-[(1-oxopropyl) amino]-3-pyridin-2-ylphényl]sulfonyl]amino]-5-[5-méthyl-1-(triphénylméthyl)-1*H*-imidazol-4-yl]-1-oxopentyl]-4-éthylpipéridine-2-carboxylate d'éthyle dans un mélange contenant 35 ml d'acide acétique et 10 ml d'eau. On concentre le milieu réactionnel sous pression réduite et on purifie le résidu par chromatographie sur colonne de gel de silice en

éluant par un mélange méthanol/dichlorométhane (5:95).
On isole 0,452 g de produit sous forme d'une huile visqueuse que l'on utilise telle quelle dans l'étape suivante.
Rendement = 83 %

4.6. chlorhydrate de [2*R*-[1(*S*), 2α, 4β]]-4-éthyl-1-[5-(5-méthyl-1*H*-imidazol-4-yl)-1-oxo-2-[[[2-[(1-oxopropyl) amino]-3-pyridin-2-ylphényl]sulfonyl]amino]pentyl] pipéridine-2-carboxylate d'éthyle

On chauffe pendant 4 heures à la température de reflux un mélange de 0,45 g (0,61 mmole) de [2*R*-[1(*S*), 2α, 4β]]-1-[2-[[[5-bromo-2-[(1-oxopropyl) amino]-3-pyridin-2-ylphényl] sulfonyl]amino]-5-[5-méthyl-1*H*-imidazol-4-yl]-1-oxopentyl]-4-éthylpipéridine-2-carboxylate d'éthyle 0,39 g (6 mmoles) de formiate d'ammonium et 50 mg de palladium sur charbon à 10 % dans 8 ml de méthanol et 0,2 ml d'acide acétique. On concentre ensuite le milieu réactionnel sous pression réduite et on purifie le résidu par chromatographie sur colonne de gel de silice en éluant par un mélange dichlorométhane/méthanol (95:5).
On obtient 0,36 g de produit sous forme d'une huile viqueuse.
Rendement = 81 %
On prépare le chlorhydrate selon la méthode décrite dans l'exemple 3.
Point de fusion = 78-84 °C
$[a]_D^{20}$ = + 91 ° (c = 0,2 ; méthanol)

<u>Exemple 5</u> (composé no 25)

chlorhydrate de [2*R*-[1(*S*), 2α, 4β]]-4-éthyl-1-[5-(5-méthyl-1*H*-imidazol-4-yl)-2-[[[3',5'-diméthyl-2-[(1-oxobutyl) amino] [1,1'-biphényl]-3-yl]sulfonyl]amino]-1-oxopentyl] pipéridine-2-carboxylate d'éthyle

5.1. sel de *N,N*-diéthyléthanamine de l'acide 2-[(3',5'-diméthyl)-2-(1-oxobutyl)amino] [1,1'-biphényle]-3-sulfonique

On chauffe pendant 2 heures à 80 °C, 2 g (7,2 mmoles) d'acide 2-amino-3',5'-diméthyl[1,1'-biphényle]-3-sulfonique dans 8,3 ml (50 mmoles) d'anhydride butyrique. On laisse la température du milieu revenir à la température ambiante et on concentre sous pression réduite. On purifie le résidu par chromatographie sur colonne RP 18 en éluant par un mélange acétonitrile/eau (2:8).
On obtient 1,5 g de produit sous forme d'un solide blanc.

On prépare le sel de triéthylamine selon la méthode décrite dans l'exemple 2 et on l'utilise tel quel dans l'étape suivante.
Rendement = 60 %

5.2. sel de N,N-diéthyléthanamine de l'acide 2-[(3',5'-diméthyl)-2-[(1-oxobutyl)(trif1uoroacétyl)amino][1,1'-biphényle]-3-sulfonique

On chauffe pendant 1 heure à la température de reflux un mélange de 1,48 g (3,3 mmoles) de sel de N,N-diéthyléthanamine de l'acide 2-[(3',5'-diméthyl)-2-(1-oxobutyl)amino] [1,1'-biphényle]-3-sulfonique et de 4,7 ml (33 mmoles) d'anhydride trifluoracétique puis on concentre le milieu réactionnel sous pression réduite.
On obtient 1,8 g de produit sous forme d'une huile visqueuse que l'on utilise telle quelle dans l'étape suivante.
Rendement = 100 %

5.3. chlorure de 2-[(3',5'-diméthyl)-2-[(1-oxobutyl) (trifluoroacétyl)amino][1,1'-biphényle]-3-sulfonyle

A une solution de 1,8 g (3,3 mmoles) de sel de N,N-diéthyléthanamine de l'acide 2-[(3',5'-diméthyl)-2-[(1-oxobutyl) (trifluoroacétyl)amino][1,1'-biphényle]-3-sulfonique dans 10 ml de dichlorométhane, on ajoute à température ambiante sous azote, 1,37 g (6,6 mmoles) de pentachlorure de phosphore, on chauffe le mélange pendant 4 heures à la température de reflux, on laisse la température du milieu réactionnel revenir à la température ambiante, on verse 100 ml d'éther, on filtre puis on concentre sous pression réduite. On purifie le résidu ainsi obtenu par chromatographie sur colonne florisil™ en éluant par du dichlorométhane.
On obtient 0,78 g de produit sous forme d'une huile visqueuse que l'on utilise telle quelle dans l'étape suivante.
Rendement = 51 %
RMN ($CDCl_3$), 200 Mhz, δ, (ppm): 8,3 (dd, 1H, J=6,5 Hz, J=0,7 Hz) ; 7,85-7,7 (m, 2H) ; 7,15-7,00 (m, 1H) ; 6,8 (s, 2H) ; 2,5-2,3 (m, 7H) ;1,75-1,5 (m, 3H) ; 0,9 (t, 3H, 6Hz)

5.4. chlorhydrate de [2*R*-[1(*S*), 2α, 4β]]-4-éthyl-1-[5-(5-méthyl-1*H*-imidazol-4-yl)-2-[[[3',5'-diméthyl-2-[(1-oxobutyl) amino][1,1'-biphényl]-3-yl]sulfonyl]amino]-1-oxopentyl] pipéridine-2-carboxylate d'éthyle

A une solution de 0,554 g (1,2 mmoles) de chlorure de 2-[(3',5'-diméthyl)-2-[(1-oxobutyl) (trifluoroacétyl)amino] [1,1'-biphényle]-3-sulfonyle dans 5 ml de dichlorométhane, on ajoute à 0 °C sous azote 0,815 g (1,2 mmoles) de chlorhydrate de [2*R*-[1(*S*), 2α, 4β]]-1-[2-amino-5-[5-méthyl-1-(triphénylméthyl)-1*H*-imidazol-4-yl]-1-oxopentyl]-4-éthylpipéridine-2-carboxylate d'éthyle et goutte à goutte 0,56 ml (4 mmoles) de triéthylamine. On laisse le milieu réactionnel sous agitation à cette température pendant 6 heures puis on le concentre sous pression réduite. On reprend le résidu dans 100 ml d'acétate d'éthyle puis on le lave successivement par 50 ml d'une solution aqueuse d'acide chlorhydrique 0,5 N, puis par 50 ml d'une solution aqueuse d'hydrogénocarbonate de sodium à 5 % puis par 50 ml d'une solution saturée de chlorure de sodium et on sèche sur sulfate de magnésium. On concentre la phase organique sous pression réduite.
On obtient 1 g de produit que l'on utilise tel quel dans l'étape suivante.
On chauffe le résidu pendant 2 heures à la température de reflux dans un mélange contenant 12 ml d'acide acétique, 6 ml de tétrahydrofurane et 6 ml d'eau puis on concentre le milieu réactionnel sous pression réduite. On purifie le résidu sur colonne de silice en éluant par un mélange méthanol/eau (5:95).
On obtient 0,475 g de produit sous forme de base sous forme d'une huile visqueuse.
Rendement = 56 %
On prépare le chlorhydrate en dissolvant 0,475 g de base dans 14 ml d'une solution d'isopropanol dans l'acide chlorhydrique 0,1 N.
Point de fusion = 135 °C
$[a]_D^{20}$ = + 118 ° (c = 0,2 ; méthanol)

Exemple 6 (composé no 34)

chlorhydrate de [2*R*-[1(*S*), 2α, 4β]]-4-éthyl-1-[5-(5-méthyl-1*H*-imidazol-4-yl)-1-oxo-2-[[[2-[(1-oxopropyl)amino]-3-thièn-2-ylphényl]sulfonyl]amino]pentyl]pipéridine-2-carboxylate d'éthyle

6.1. sel de *N,N*-diéthyléthanamine de l'acide 2-[bis(1-oxopropyl)amino]-3-thièn-2-ylbenzènesulfonique

On dissout 1,9 g (7,8 mmoles) d'acide 2-amino-3-thièn-2-ylbenzènesulfonique dans 10 ml de dichlorométhane, on ajoute 1,3 ml (9,4 mmoles) de triéthylamine et on évapore à sec. On dissout alors le sel obtenu dans 15 ml d'anhydride propionique, on chauffe le mélange pendant 8 heures à 150 °C et on évapore à sec. On purifie le résidu par chromatographie sur colonne de gel de silice en éluant par un mélange dichlorométhane/éthanol/triéthylamine (98:2:0,001).
On obtient 2,76 g de produit après recristallisation dans l'acétate d'éthyle.
Rendement = 79 %

6.2. chlorure de 2-[bis(l-oxopropyl)amino]-3-thièn-2-yl benzènesulfonyle

A une solution de 2,8 g (6,1 mmoles) de sel de N,N-diéthyléthanamine de l'acide 2-[bis(1-oxopropyl)amino]-3-thièn-2-ylbenzènesulfonique dans 6 ml de dichlorométhane, à 0 °C, on ajoute 1,3 g (6,1 mmoles) de pentachlorure de phosphore et on laisse la température du mélange revenir à la température ambiante. On chauffe ensuite le mélange pendant 5 heures à la température de reflux puis on laisse la température revenir à l'ambiante. On ajoute de l'éther, on filtre et on précipite le résidu par un mélange éther/pentane (1:1). On filtre, on concentre le filtrat sous pression réduite et on le purifie par chromatographie sur colonne de florisil™. On obtient 2,2 g de produit que l'on cristallise dans un mélange éther/pentane.
On obtient 1,5 g de produit sous forme de cristaux blancs.
Rendement = 55 %
Point de fusion = 113,6 °C

6.3. [2*R*-[1(*S*), 2α, 4β]]-1-[2-[[[2-[bis(1-oxopropyl)amino]-3-thièn-2-ylphényl]sulfonyl]amino]-5-[5-méthyl-1-(triphénylméthyl)-1*H*-imidazol-4-yl]-1-oxopentyl]-4-éthylpipéridine-2-carboxylate d'éthyle

On dissout 0,9 g (2 mmoles) de chlorure de 2-[bis(1-oxopropyl)amino]-3-thièn-2-ylbenzènesulfonyle dans 8 ml de dichlorométhane et on place le mélange à 0 °C. On ajoute alors 1,3 g (2 mmoles) de chlorhydrate de [2*R*-[1(*S*), 2α, 4β]]-1-[2-amino-5-[5-méthyl-1-(triphénylméthyl)-1H-imidazol-4-yl]-1-oxopentyl]-4-éthylpipéridine-2-carboxylate d'éthyle et goutte à goutte 0,9 ml de triéthylamine. On laisse sous agitation à cette température pendant une nuit. On

concentre sous pression réduite, on reprend le résidu par 100 ml d'acétate d'éthyle et on lave successivement par 50 ml d'une solution d'acide chlorhydrique 1 N, par 50 ml d'une solution saturée en hydrogénocarbonate de sodium puis par 50 ml d'une solution saturée en chlorure de sodium. On sèche sur sulfate de magnésium et on concentre sous pression réduite. On purifie le résidu par chromatographie sur colonne de gel de silice en éluant par un mélange dichlorométhane/méthanol (98:2).
On obtient 2 g de produit que l'on utilise tel quel dans l'étape suivante.

6.4. chlorhydrate de [2*R*-[1(*S*), 2α, 4β]]-4-éthyl-1-[5-(5-méthyl-1*H*-imidazol-4-yl)-1-oxo-2-[[[2-[(1-oxopropyl) amino]-3-thièn-2-ylphényl]sulfonyl]amino]pentyl] pipéridine-2-carboxylate d'éthyle

A 1,9 g (2 mmoles) de [2*R*-[1(*S*), 2α, 4β]]-1-[2-[[[2-[bis(1-oxopropyl)amino]-3-thièn-2-ylphényl]sulfonyl]amino]-5-[5-méthyl-1-(triphénylméthyl)-1*H*-imidazol-4-yl]-1-oxopentyl]-4-éthylpipéridine-2-carboxylate d'éthyle, on ajoute 100 ml d'acide acétique et 20 ml d'eau et on chauffe le mélange à la température de reflux pendant 6 heures. On évapore à sec, on reprend le résidu par 100 ml d'acétate d'éthyle et on lave par 10 ml d'une solution saturée en hydrogénocarbonate de sodium puis par 10 ml d'une solution saturée en chlorure de sodium, on sèche sur sulfate de magnésium et on évapore à sec. On purifie le résidu par chromatographie sur colonne de gel de silice en éluant par un gradient dichlorométhane/méthanol.
On obtient 1,1 g de produit sous forme de base.
On prépare le chlorhydrate dans 10 ml d'une solution d'isopropanol dans l'acide chlorhydrique 0,1 N et on concentre de nouveau sous pression réduite. On purifie ce dernier résidu sur colonne RP 18 en éluant par un mélange acétonitrile: eau (1:1)
Après lyophylisation on obtient 0,939 g de produit sous forme de chlorhydrate.
Rendement = 67 %
Point de fusion = 151 °C
$[a]_D^{20}$ = + 107 ° (c = 0,2 ; méthanol)

<u>Exemple 7</u> (composé no 42)

chlorhydrate de [2*R*-[1(*S*), 2α, 4β]]-1-[2-[[[2-(acétylamino)-3-cyclopentylphényl]sulfonyl]aminol-5-(5-méthyl-1*H*-imidazol-4-yl)-1-oxopentyl]-4-éthylpipéridine-2-carboxylate d'éthyle

7.1. sel de *N,N*-diéthyléthanamine de l'acide 2-(diacétylamino)-3-cyclopentylbenzènesulfonique

On chauffe pendant 48 heures à la température de reflux 3,42 g (10 mmoles) de sel de N,N-diéthyléthanamine de l'acide 2-amino-3-cyclopentylbenzènesulfonique en solution dans 100 ml de chlorure d'acétyle puis on concentre le milieu réactionnel sous pression réduite.
On obtient 4,3 g de produit sous forme d'une huile que l'on utilise telle quelle dans l'étape suivante.
Rendement = 100 %

7.2. chlorure de 2-(diacétylamino)-3-cyclopentylbenzène sulfonyle

On chauffe pendant 2,5 heures à la température de reflux une solution de 1,55 g (3,6 mmoles) d'acide 2-(diacétylamino)-3-cyclopentylbenzènesulfonique et de 1,12 g (5,4 mmoles) de pentachlorure de phosphore dans 10 ml de dichlorométhane, puis on concentre le milieu réactionnel sous pression réduite. On purifie le résidu ainsi obtenu par chromatographie sur colonne de florisil™ en éluant par du dichlorométhane.
On obtient 0,62 g de produit sous forme d'une huile visqueuse que l'on utilise telle quelle dans l'étape suivante.
Rendement = 50 %

7.3. [2*R*-[1(*S*), 2α, 4β]]-1-[2-[[[2-(diacétylamino)-3-cyclopentylphényl]sulfonyl]amino]-5-[5-méthyl-1-(triphénylméthyl)-1*H*-imidazol-4-yl]-1-oxopentyl]-4-éthylpipéridine-2-carboxylate d'éthyle

A un mélange de 0,77 g (1,2 mmoles) de chlorhydrate de [2*R*-[1(*S*), 2α, 4β]]-1-[2-amino-5-[5-méthy1-1-(triphénylméthyl)-1*H*-imidazol-4-yl]-1-oxopentyl]-4-éthylpipéridine-2-carboxylate d'éthyle et de 0,37 ml (2,64 mmoles) de triéthylamine dans 5 ml de dichlorométhane, on ajoute goutte à goutte, à 0 °C sous atmosphère d'azote, 0,35 g (1 mmole) de chlorure de 2-(diacétylamino)-3-cyclopentylbenzènesulfonyle en solution dans 1 ml de dichlorométhane. On laisse le milieu réactionnel sous agitation à cette température pendant 6 heures puis on le concentre sous pression réduite. On reprend le résidu dans 50 ml d'acétate d'éthyle puis on le lave successivement par 50 ml d'une solution aqueuse d'acide chlorhydrique 0,5 N, puis par 50 ml d'une solution saturée d'hydrogénocarbonate de sodium puis par 50 ml

d'une solution saturée de chlorure de sodium et on sèche sur sulfate de magnésium. On concentre la phase organique sous pression réduite.
On utilise le résidu tel quel dans l'étape suivante.

7.4. chlorhydrate de [2*R*-[1(*S*), 2α, 4β]]-1-[2-[[[2-(acétylamino)-3-cyclopentylphényl]sulfonyl]amino]-5-(5-méthyl-1*H*-imidazol-4-yl)-l-oxopentyl]-4-éthylpipéridine-2-carboxylate d'éthyle

On chauffe pendant 48 heures à la température de reflux du [2*R*-[1(*S*), 2α, 4β]]-1-[2-[[[2-(diacétylamino)-3-cyclopentyl phényl]sulfonyl]amino]-5-[5-méthyl-1-(triphénylméthyl)-1*H*-imidazol-4-yl]-1-oxopentyl]-4-éthylpipéridine-2-carboxylate d'éthyle dans un mélange contenant 8 ml d'acide acétique, 4 ml de tétrahydrofurane et 4 ml d'eau puis on concentre le milieu réactionnel sous pression réduite. On reprend le résidu dans 18 ml d'une solution d'isopropanol dans l'acide chlorhydrique 0,1 N et on concentre de nouveau sous pression réduite. On purifie ce dernier résidu sur colonne RP 18 en éluant par un mélange acétonitrile:eau (6:4).
On obtient 0,31 g de produit sous forme de chlorhydrate.
Rendement = 50 %
Point de fusion = 140 °C
$[a]_D^{20}$ = + 74,5 ° (c = 0,25 ; méthanol)

Légende du tableau :

*dans la colonne "Sel" :*
"chlor." représente un chlorhydrate l'absence de mention correspond au produit sous forme de base
*dans la colonne "$[\alpha]_D^{20}$"* : c = 0,2 sauf indication contraire ; solvant = méthanol.

Tableau

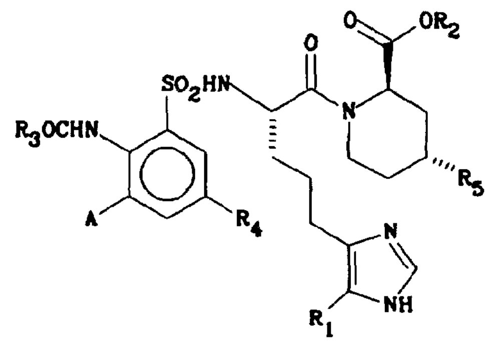

(I)

| No | $R_1$ | $R_2$ | $R_3$ | $R_4$ | $R_5$ | A | Sel | F (° C) | $[\alpha]_D^{20}$ (°) |
|---|---|---|---|---|---|---|---|---|---|
| 1 | $-CH_3$ | $-C_2H_5$ | $-CH_3$ | -H | $-C_2H_5$ | 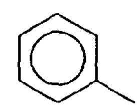 | chlor. | 136-141 | + 128,8 (c=0,25) |
| 2 | $-CH_3$ | H | $-CH_3$ | -H | $-C_2H_5$ | 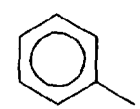 | chlor. | 165-170 | + 92,4 (c=0,25) |
| 3 | $-CH_3$ | $-C_2H_5$ | $-C_2H_5$ | -H | $-C_2H_5$ | 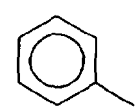 | chlor. | 104-105 | + 105 |

| No | $R_1$ | $R_2$ | $R_3$ | $R_4$ | $R_5$ | A | Sel | F (° C) | $[\alpha]_D^{20}$ (°) |
|---|---|---|---|---|---|---|---|---|---|
| 4 | $-CH_3$ | $-C_2H_5$ | $-C_3H_7$ | -H | $-C_2H_5$ | 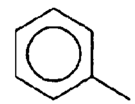 | chlor. | 95 | + 109,5 |
| 5 | -H | $-C_2H_5$ | $-CH_3$ | -H | $-CH_3$ | 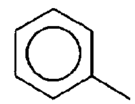 | chlor. | 125 | + 103 |
| 6 | -H | -H | $-CH_3$ | -H | $-CH_3$ | 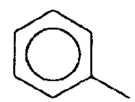 | chlor. | 168-175 | + 68,4 (c=0,25) |
| 7 | -H | $-C_2H_5$ | $-C_2H_4OCH_3$ | -Br | $-C_2H_5$ | 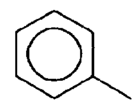 | chlor. | 120 | + 52,5 |
| 8 | -H | $-C_2H_5$ | $-C_2H_4OCH_3$ | -H | $-C_2H_5$ | 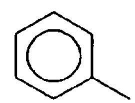 | chlor. | 74 | + 60 |
| 9 | -H | -H | $-C_2H_4OCH_3$ | -H | $-C_2H_5$ | 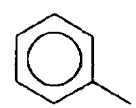 | chlor. | 132 | + 59 |
| 10 | -H | $-C_2H_5$ | $-C_3H_7$ | -Br | $-C_2H_5$ | 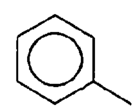 | chlor. | 121 | + 83 |

| No | $R_1$ | $R_2$ | $R_3$ | $R_4$ | $R_5$ | A | Sel | F (° C) | $[\alpha]_D^{20}$ (°) |
|---|---|---|---|---|---|---|---|---|---|
| 11 | -H | $-C_2H_5$ | $-C_3H_7$ | -H | $-C_2H_5$ | 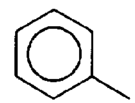 | chlor. | 125-127 | + 124 |
| 12 | -H | -H | $-C_3H_7$ | -H | $-C_2H_5$ | 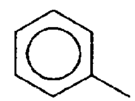 | chlor. | 141 | + 116 |
| 13 | -H | $-C_2H_5$ | $-CH_2O(C_2H_4O)_2CH_3$ | -H | $-C_2H_5$ | 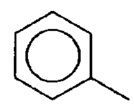 | chlor. | 74 | + 77 |
| 14 | $-CH_3$ | $-C_2H_5$ | $-CH_3$ | -H | $-C_2H_5$ | 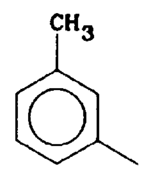 | chlor. | 134 | + 113 |
| 15 | $-CH_3$ | $-C_2H_5$ | $-C_2H_5$ | -H | $-C_2H_5$ | 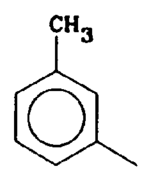 | chlor. | 140 | + 115 |

| No | $R_1$ | $R_2$ | $R_3$ | $R_4$ | $R_5$ | A | Sel | F (° C) | $[\alpha]_D^{20}$ (°) |
|---|---|---|---|---|---|---|---|---|---|
| 16 | $-CH_3$ | $-C_2H_5$ | $-C_3H_7$ | -H | $-C_2H_5$ | 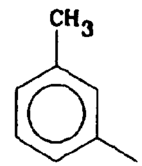 | chlor. | 120 | + 112,5 |
| 17 | $-CH_3$ | $-C_2H_5$ | $-C_2H_5$ | -H | $-C_2H_5$ | 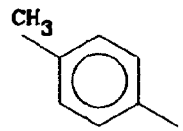 | chlor. | 128 | + 94 |
| 18 | $-CH_3$ | $-C_2H_5$ | $-C_2H_5$ | -H | $-C_2H_5$ | 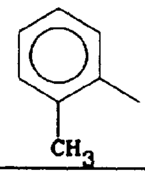 | chlor. | 130 | + 110 |
| 19 | $-CH_3$ | $-C_2H_5$ | $-CH_3$ | -H | $-C_2H_5$ | 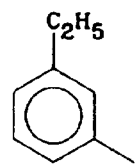 | chlor. | 136 | + 110 |
| 20 | $-CH_3$ | $-C_2H_5$ | $-C_2H_5$ | -H | $-C_2H_5$ | 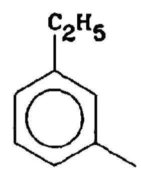 | chlor. | 108 | + 103 |

| No | $R_1$ | $R_2$ | $R_3$ | $R_4$ | $R_5$ | A | Sel | F (° C) | $[\alpha]_D^{20}$ (°) |
|---|---|---|---|---|---|---|---|---|---|
| 21 | $-CH_3$ | $-C_2H_5$ | $-C_2H_5$ | -H | $-C_2H_5$ | 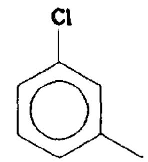 | chlor. | 154 | + 103 |
| 22 | $-CH_3$ | $-C_2H_5$ | $-C_2H_5$ | -H | $-C_2H_5$ | 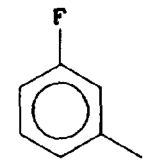 | chlor. | 126 | + 54 |
| 23 | $-CH_3$ | $-C_2H_5$ | $-C_3H_7$ | -H | $-C_2H_5$ | 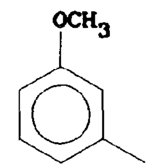 | chlor. | 65 | + 104,5 |
| 24 | $-CH_3$ | $-C_2H_5$ | $-C_2H_5$ | -H | $-C_2H_5$ | 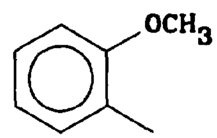 | chlor. | 128 | + 54 |
| 25 | $-CH_3$ | $-C_2H_5$ | $-C_3H_7$ | -H | $-C_2H_5$ | 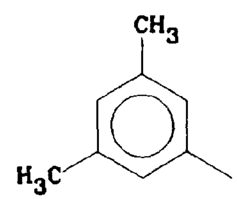 | chlor. | 135 | + 118 |

| No | $R_1$ | $R_2$ | $R_3$ | $R_4$ | $R_5$ | A | Sel | F (° C) | $[\alpha]_D^{20}$ (°) |
|---|---|---|---|---|---|---|---|---|---|
| 26 | $-CH_3$ | $-C_2H_5$ | $-C_2H_5$ | -H | $-C_2H_5$ | 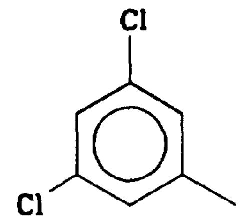 | chlor. | 130 | + 125 |
| 27 | -H | $-C_2H_5$ | $-CH_3$ | -H | $-C_2H_5$ | 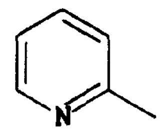 | chlor. | 131 | + 42,5 |
| 28 | -H | -H | $-CH_3$ | -H | $-C_2H_5$ | 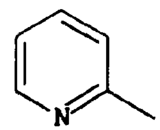 | chlor. | 172 | + 57 |
| 29 | $-CH_3$ | $-C_2H_5$ | $-C_2H_5$ | -H | $-C_2H_5$ | 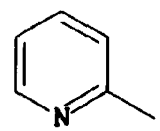 | chlor. | 78-84 | 91 |
| 30 | $-CH_3$ | $-C_2H_5$ | $-CH_3$ | -H | $-C_2H_5$ | 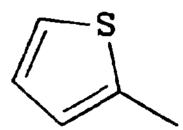 | chlor. | 178,2 | + 97,6 (c=0,25) |

| No | $R_1$ | $R_2$ | $R_3$ | $R_4$ | $R_5$ | A | Sel | F (° C) | $[\alpha]_D^{20}$ (°) |
|---|---|---|---|---|---|---|---|---|---|
| 31 | $-CH_3$ | -H | $-CH_3$ | -H | $-C_2H_5$ | 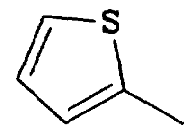 | chlor. | 150-155 | + 111 (c=0,55) |
| 32 | -H | $-C_2H_5$ | $-CH_3$ | -H | $-C_2H_5$ | 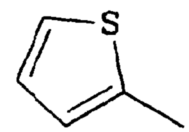 | chlor. | 90 | + 73 |
| 33 | -H | -H | $-CH_3$ | -H | $-C_2H_5$ | 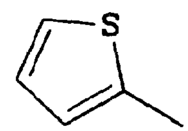 | chlor. | 150 | + 82,5 |
| 34 | $-CH_3$ | $-C_2H_5$ | $-C_2H_5$ | -H | $-C_2H_5$ | 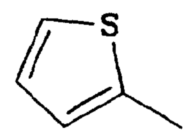 | chlor. | 151 | + 107 |
| 35 | $-CH_3$ | $-C_2H_5$ | $-C_3H_7$ | -H | $-C_2H_5$ | 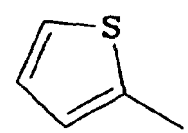 | chlor. | 124 | + 101 |

| No | $R_1$ | $R_2$ | $R_3$ | $R_4$ | $R_5$ | A | Sel | F (° C) | $[\alpha]_D^{20}$ (°) |
|---|---|---|---|---|---|---|---|---|---|
| 36 | $-CH_3$ | $-C_2H_5$ | $-C_3H_7$ | -H | $-C_2H_5$ | 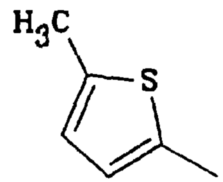 | chlor. | 110 | + 52,6 |
| 37 | $-CH_3$ | $-C_2H_5$ | $-C_2H_5$ | -H | $-C_2H_5$ | 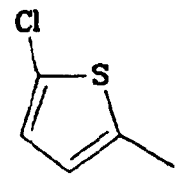 | chlor. | 132 | + 63 |
| 38 | $-CH_3$ | $-C_2H_5$ | $-C_3H_7$ | -H | $-CH_3$ | 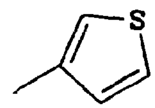 | chlor. | 130-132 | + 132 |
| 39 | $-CH_3$ | $-C_2H_5$ | $-C_2H_5$ | -H | $-C_2H_5$ | 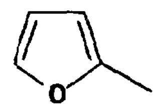 | chlor. | 128-134 | + 57 |
| 40 | -H | $-C_2H_5$ | $-CH_3$ | -H | $-C_2H_5$ | 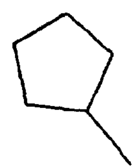 | chlor. | 124 | + 78 |

| No | $R_1$ | $R_2$ | $R_3$ | $R_4$ | $R_5$ | A | Sel | F (° C) | $[\alpha]_D^{20}$ (°) |
|---|---|---|---|---|---|---|---|---|---|
| 41 | -H | -H | $-CH_3$ | -H | $-C_2H_5$ | 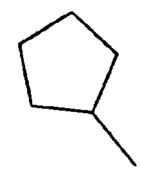 | chlor. | 149 | + 102 |
| 42 | $-CH_3$ | $-C_2H_5$ | $-CH_3$ | -H | $-C_2H_5$ | 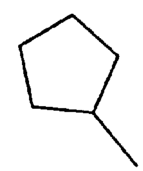 | chlor. | 140 | + 74,5 |
| 43 | $-CH_3$ | -H | $-CH_3$ | -H | $-C_2H_5$ | 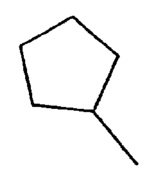 | chlor. | 160 | + 81,5 |
| 44 | $-CH_3$ | $-C_2H_5$ | $-C_2H_5$ | -H | $-C_2H_5$ | 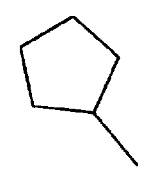 | chlor. | 118 | + 93 |
| 45 | -H | $-C_2H_5$ | $-C_3H_7$ | -H | $-C_2H_5$ | 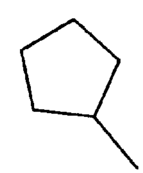 | chlor. | 125 | + 73,5 |

| No | $R_1$ | $R_2$ | $R_3$ | $R_4$ | $R_5$ | A | Sel | F (° C) | $[\alpha]_D^{20}$ (°) |
|---|---|---|---|---|---|---|---|---|---|
| 46 | $-CH_3$ | $-C_2H_5$ | $-C_3H_7$ | -H | $-CH_3$ | 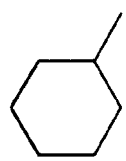 | chlor. | 132 | + 104 |
| 47 | -H | $-C_2H_5$ | $-C_3H_7$ | -H | $-C_2H_5$ | 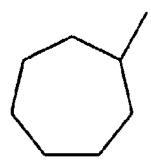 | chlor. | 120 | + 77 |

Les composés de l'invention ont fait l'objet d'études pharmacologiques qui ont mis en évidence leurs propriétés

antithrombotiques et leur intérêt comme substances à activité thérapeutique.

1. Détermination des constantes d'inhibition (Ki) vis à vis de la thrombine

Sur une microplaque de 96 puits, on dépose dans chaque puits 25 µl d'une solution de composé à tester (on étudie 7 concentrations), 50 µl d'une solution de substrat chromogène (on étudie 2 concentrations ; S2238 Chromogénix™) en solution dans du tampon Tris à pH 7,5 (Tris 50 mM, NaCl 100 mM et BSA 0,1 %) et finalement 25 µl d'une solution de thrombine à 300 U/ml. On suit la libération de 4-nitroaniline à 405 nm à l'aide d'un lecteur de plaques.
On détermine le $K_i$ par la méthode de Dixon.
Les composés de l'invention sont des inhibiteurs de la thrombine et leur $K_i$ est compris entre 0,001 et 100 µM.

2. Coagulation du plasma de rat par la thrombine humaine *ex-vivo*

On traite des rats mâles CD pesant 150 à 200 g avec le composé à tester ou avec le véhicule, par voie i.v., orale ou sous-cutanée. Ensuite on anesthésie les animaux au Nembutal™ (60 mg/kg ; 0,1 ml/kg), on prélève le sang sur du citrate trisodique à 3,8% (1 vol/9 vol de sang) au niveau du sinus rétro-orbital et on prépare le plasma par centrifugation à 3600 g pendant 15 minutes à la température ambiante. On incube alors à 37 °C, 200 µl de plasma avec 200 µl d'une solution de thrombine humaine, la concentration finale en thrombine humaine étant de 0,75 unités NIH/ml et on note le temps de coagulation. L'effet anticoagulant est exprimé par la dose qui augmente le temps de coagulation de 100 %. Ils inhibent la coagulation du plasma de rat à des doses de 0,01 à 5 mg/kg i.v. Ils sont également actifs par les voies orale et sous-cutanée.

3. Agrégation des plaquettes de lapin induite par la thrombine humaine.

On prélève le sang par ponction cardiaque sur du citrate trisodique à 3,8 % (1 vol/9 vol de sang). On le centrifuge à 250 g pendant 10 minutes. On prélève le plasma riche en plaquettes ($P_3P$) ainsi obtenu et on réalise la numération des plaquettes.
Au $P_3P$, on ajoute 2 ng/ml de prostacycline en solution dans du tampon tris à pH 9,0 glacé. On centrifuge à 110 g, pendant 10 minutes et on décante. On ajoute à nouveau de la prostacycline, en solution dans de l'hydroxyde de sodium 50 mM à pH 12, de manière à avoir une concentration finale de 200 ng/ml. On centrifuge à nouveau le $P_3P$ à 800 g pendant 10 minutes. On élimine le plasma pauvre en plaquettes et on met le culot en suspension dans un volume de tyrode contenant 200 ng/ml de prostacycline, volume égal au volume initial de $P_3P$. On centrifuge cette suspension à 800 g pendant 10 minutes. On recommence une seconde fois et dans les mêmes conditions, la mise en suspension du culot et la centrifugation. On remet le culot final en suspension dans une solution de tyrode sans prostacycline et on laisse au repos pendant 2 heures pour permettre l'élimination complète de la prostacycline. On induit l'agrégation de ces plaquettes avec de la thrombine humaine à la concentration finale de 0,3 unités NIH/ml. On enregistre les variations de densité optique au moyen d'un agrégomètre à 4 canaux. On ajoute le composé à tester ou son véhicule à la suspension de plaquettes (volume maximal ajouté de 3 µl), 2 minutes avant l'addition de thrombine. On détermine la concentration qui inhibe l'agrégation de 50 % ($CI_{50}$).

Les composés de l'invention peuvent être utiles dans toutes les indications cliniques liées à la thrombose ou dans celles où des complications thrombotiques pourraient intervenir.

A cet effet ils peuvent être présentés sous toutes formes appropriées à l'administration orale, parentérale ou intraveineuse, telles que comprimés, dragées, gélules, capsules, suspensions ou solutions buvables ou injectables, etc. en association avec des excipients convenables. Toutes ces formes sont dosées pour permettre une administration de 1 à 1000 mg par jour et par patient, en une ou plusieurs doses.

**Revendications**

**1.** Composés répondant à la formule (I)

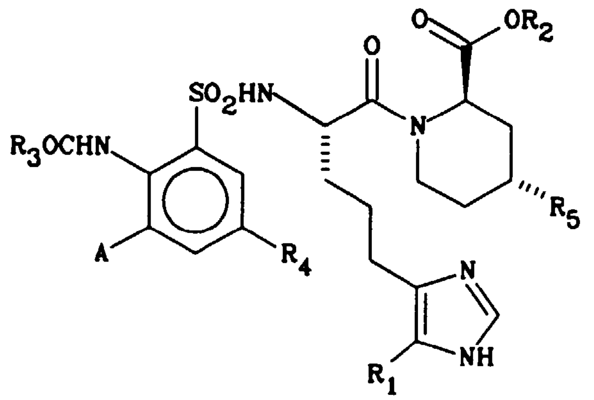

(I)

dans laquelle

$R_1$ représente soit un atome d'hydrogène, soit un groupe ($C_1$-$C_4$) alkyle,
$R_2$ représente soit un atome d'hydrogène, soit un groupe ($C_1$-$C_4$)alkyle droit ou ramifié,
$R_3$ représente soit un groupe ($C_1$-$C_7$)alkyle droit ou ramifié, soit un groupe $-(CH_2)_nOCH_3$ (où n est 1, 2 ou 3), soit un groupe $-CH_2O(C_2H_4O)_mCH_3$ (où m est 1, 2 ou 3),
$R_4$ représente soit un atome d'hydrogène, soit un atome d'halogène,
$R_5$ représente un groupe ($C_1$-$C_4$)alkyle droit ou ramifié et A représente soit un groupe phényle éventuellement substitué par un ou plusieurs substituants choisis parmi les atomes d'halogènes et les groupes ($C_1$-$C_4$)alkyle droit ou ramifié, ($C_1$-$C_4$)alcoxy droit ou ramifié et trifluorométhyle, soit un hétérocycle choisi parmi les groupes pyridinyle, thiényle, furyle, éventuellement substitués comme ci-dessus, soit un groupe cyclo($C_5$-$C_8$)alkyle, sous forme de base libre ou de sels d'addition à des acides pharmaceutiquement acceptables.

**2.** Composés selon la revendication 1 caractérisés en ce que $R_3$ représente un groupe ($C_1$-$C_7$)alkyle droit ou ramifié et A représente un hétérocycle choisi parmi les groupes pyridinyle, thiényle, furyle, éventuellement substitués par un ou plusieurs substituants choisis parmi les atomes d'halogènes et les groupes ($C_1$-$C_4$)alkyle droit ou ramifié, ($C_1$-$C_4$)alcoxy droit ou ramifié et trifluorométhyle.

**3.** Composés selon l'une quelconque des revendications 1 et 2 caractérisés en ce que la configuration préférentielle du groupe pipéridinyle est [2*R*, 4*R*].

**4.** Composés selon l'une quelconque des revendications 1 à 3 caractérisés en ce que la configuration de la partie acide aminé centrale

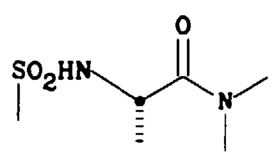

est [*S*].

**5.** Procédé de préparation des composés selon la revendication 1 caractérisé en ce que l'on fait réagir un acide sulfonique de formule (II)

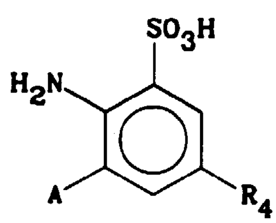

(II)

dans laquelle A et $R_4$ sont tels que définis dans la revendication 1, avec un chlorure d'acide de formule (III)

$R_3COCl$ (III)

dans laquelle $R_3$ est tel que défini dans la revendication 1, puis on ajoute de la triéthylamine pour obtenir un sel de triéthylamine de formule (IV)

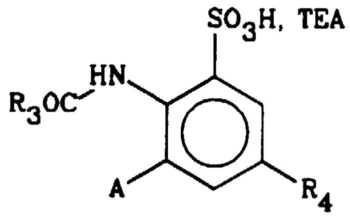

(IV)

que l'on fait réagir avec de l'anhydride trifluoroacétique et on obtient un composé de formule (V)

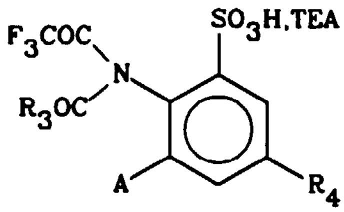

(V)

sur lequel on fait agir du pentachlorure de phosphore pour obtenir un composé de formule (VI)

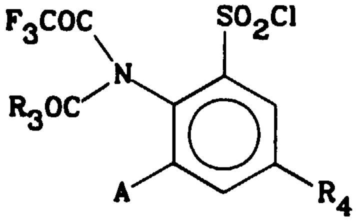

(VI)

que l'on condense avec le chlorhydrate de l'amine de formule (VII)

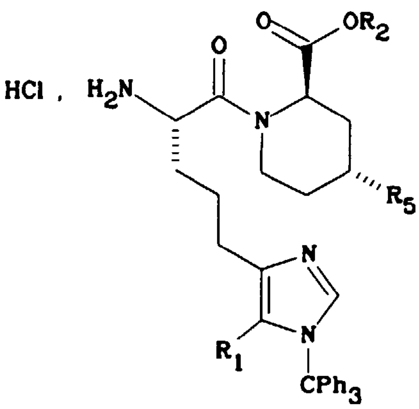

(VII)

dans laquelle $R_1$, $R_2$ et $R_5$ sont tels que définis dans la revendication 1, puis on traite en milieu acide pour obtenir un composé de formule (I) (dans laquelle $R_2$ représente un groupe ($C_1$-$C_4$)alkyle droit ou ramifié) que l'on saponifie si l'on souhaite obtenir un composé de formule (I) dans laquelle $R_2$ représente un atome d'hydrogène.

**6.** Procédé de préparation des composés selon la revendication 1 de formule (Ia)

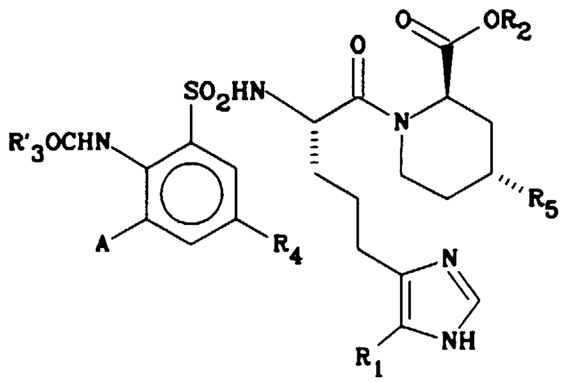

(Ia)

dans laquelle R'$_3$ représente un groupe ($C_1$-$C_7$)alkyle droit ou ramifié, caractérisé en ce qu'on fait réagir un composé de formule (II) avec un chlorure d'acide de formule (IIIa)

$$R'_3COCl \qquad (IIIa)$$

ou avec un anhydride de formule (IIIb)

$$(R'_3CO)_2O \qquad (IIIb)$$

et on obtient un imide symétrique dont on prépare le sel de triéthylamine de formule (Va)

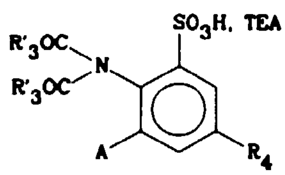

(Va)

que l'on traite par du pentachlorure de phosphore pour obtenir un composé de formule (VIa)

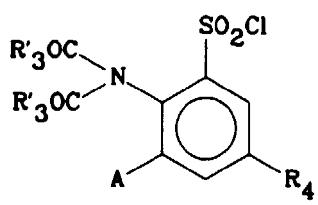

(VIa)

sur lequel on fait réagir le chlorhydrate de l'amine de formule (VII) puis on traite en milieu acide et on obtient un composé de formule (Ia) (dans laquelle $R_2$ représente un groupe ($C_1$-$C_4$)alkyle droit ou ramifié) que l'on saponifie si on souhaite obtenir un composé de formule (Ia) dans laquelle $R_2$ représente un atome d'hydrogène.

**7.** Procédé de préparation des composés de formule (Ia) caractérisé en ce que l'on traite un composé de formule (IIb)

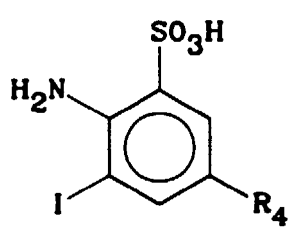

(IIb)

dans laquelle $R_4$ représente un atome d'halogène avec de la triéthylamine et on fait réagir le sel ainsi obtenu avec un chlorure d'acide de formule (IIIa)

$$R'_3COCl \qquad (IIIa)$$

dans laquelle R'$_3$ représente un groupe ($C_1$-$C_7$)alkyle droit ou ramifié, et on obtient un composé de formule (IVb)

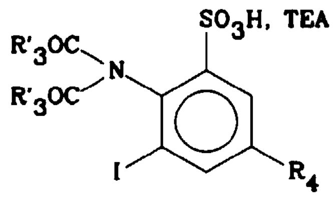

(IVb)

à partir duquel on prépare un composé de formule (VIb)

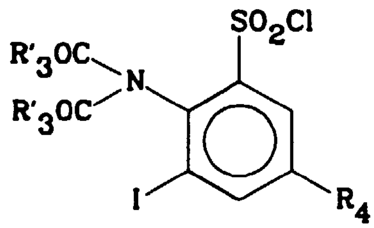

(VIb)

par action du pentachlorure de phosphore puis on fait réagir le composé (VIb) avec le chlorhydrate de l'amine de formule (VII) puis on traite en milieu basique et on obtient un composé de formule (VIII)

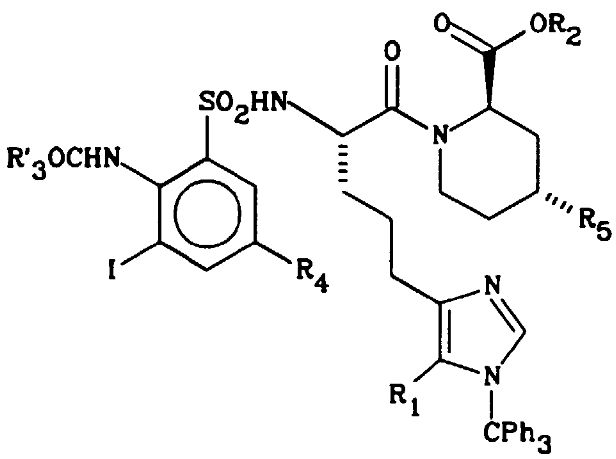

(VIII)

que l'on fait réagir avec un composé de formule (IX)

$$ASn(R)_3 \qquad (IX)$$

dans laquelle A est tel que défini dans la revendication 1 et R est un groupe $(C_1\text{-}C_4)$alkyle pour former un composé de formule (X)

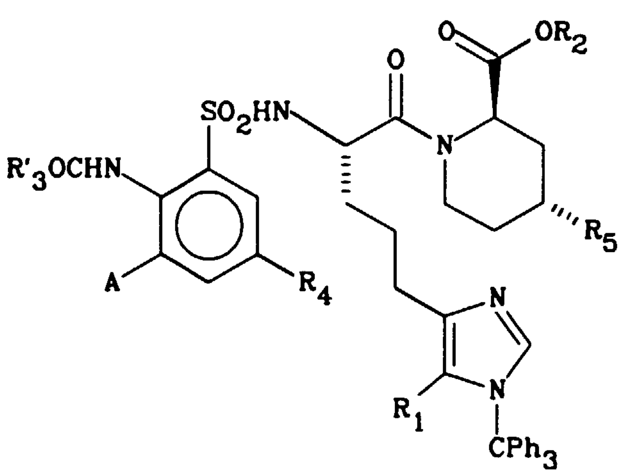

(X)

que l'on chauffe à la température de reflux en milieu acide, et on obtient un composé de formule (Ia) dans laquelle $R_4$ représente un atome d'halogène, composé que l'on soumet à une hydrogénolyse si l'on souhaite obtenir un

composé de formule (Ia) dans laquelle $R_4$ est un atome d'hydrogène, puis que l'on saponifie si l'on veut préparer un composé de formule (Ia) dans laquelle $R_2$ représente un atome d'hydrogène.

**7.** Composés de formule (XI)

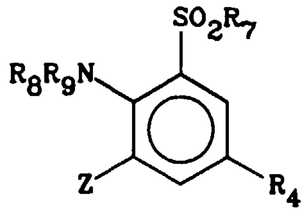

(XI)

dans laquelle soit $R_6$ représente un atome d'hydrogène ou un groupe trifluoroacétyle, $R_9$ un groupe $-COR_3$ (où $R_3$ est tel que défini précédemment) et $R_7$ un groupe hydroxy, soit $R_8$ représente un groupe trifluoroacétyle, $R_9$ un groupe $-COR_3$ et $R_7$ un atome de chlore, soit $R_8$ et $R_9$ représentent chacun un groupe $-COR'_3$ (où $R'_3$ est tel que défini précédemment) et $R_7$ un atome de chlore ou un groupe hydroxy,
Z représente un atome d'iode ou un groupe phényle éventuellement substitué par un ou plusieurs substituants choisis parmi les atomes d'halogènes et les groupes $(C_1-C_4)$alkyle droit ou ramifié, $(C_1-C_4)$alcoxy droit ou ramifié et trifluorométhyle, soit un hétérocycle choisi parmi les groupes pyridinyle, thiényle, furyle, éventuellement substitués comme ci-dessus, soit un groupe cyclo$(C_5-C_8)$alkyle et
$R_4$ est tel que défini dans la revendication 1,
utiles comme intermédiaires dans la synthèse des composés selon la revendication 1.

**9.** Médicament caractérisé en ce qu'il contient un composé selon l'une quelconque des revendications 1 à 4.

**10.** Composition pharmaceutique caractérisée en ce qu'elle contient un composé selon l'une quelconque des revendications 1 à 4 en association avec tout excipient pharmaceutiquement acceptable.